# EUROPEAN PATENT SPECIFICATION

(11) **EP 1 704 144 B1**
(45) Date of publication and mention of the grant of the patent: **07.02.2007**
(21) Application number: 04821059.5
(22) Date of filing: 17.12.2004
(51) Int. Cl.: C07D 239/42

(54) **A METHOD OF PREPARATION OF THE HEMI-CALCIUM SALT OF (E)-7- [4-(4-FLUOROPHENYL)-6-ISOPROPYL-2- [ME THYL(METHYLSULFONYL)AMINO]PYRIMIDIN-5-YL](3R,5S)-3,5-DIHYDROXY-6-HEPTENOIC ACID**
VERFAHREN ZUR HERSTELLUNG DES HEMICALCIUMSALZES VON (E)-7-[4-(4-FLUORPHENYL)-6-ISOPROPYL-2-[METHYL(METHYLSULFONYL)AMINO]PYRIMIDIN-5-YL](3R,5S)-3,5-DIHYDROXY-6-HEPTENSÄURE
PROCEDE DE PREPARATION DU SEL MOITIE CALCIUM DE L'ACIDE (E)-7-[4-(4-FLUOROPHENYL)-6-ISOPROPYL-2- [METHYL(METHYLSULFONYL)AMINO]PYRIMIDIN-5-YL](3R,5S)-3,5-DIHYDROXY-6-HEPTENOIQUE

(30) Priority: 16.01.2004 CZ 200486
(43) Date of publication of application: 27.09.2006
(73) Proprietor: Zentiva, a.s., 102 37 Praha 10 (CZ)
(72) Inventor: SEBEK, Pavel, 161 00 Praha 6 (CZ); RADL, Stanislav, 143 00 Praha 2 (CZ); STACH, Jan, 190 00 Praha 9 - Ujezd nad Lesy (CZ)
(74) Representative: Jirotkova, Ivana
(86) International application number: PCT/CZ2004/000088
(87) International publication number: WO 2005/068435

(56) References cited:
- EP-A- 0 521 471
- WO-A-00/42024
- WO-A-03/016317

## Description

### Technical Field

The invention concerns a new method of preparation of the hemi-calcium salt of (*E*)-7-[4-(4-fluorophenyl)-6-isopropyl-2-[methyl(methylsufonyl)amino]pyrimidin-5-yl](3*R*,5*S*)-3,5-dihydroxy-6-heptenoic acid known under the INN name rosuvastatin, formula I.

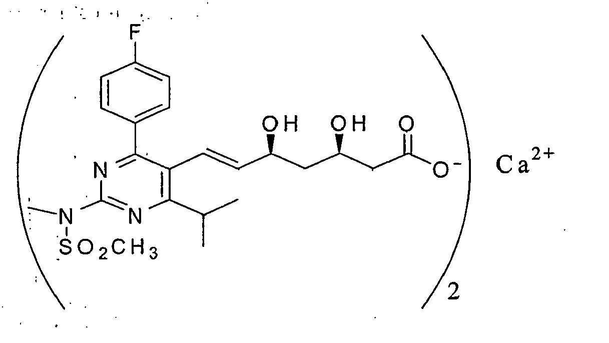

The mentioned medicament is a prominent representative of hypolipidemic and hypocholesteric pharmaceuticals.

### Background Art

Rosuvastatin is produced according to the published patent (EP 521471) usually from the sodium salt of (*E*)-7-[4-(4-fluorophenyl)-6-isopropyl-2-[methyl(methylsulfonyl)amino]-pyrimidin-5-yl] (3*R*,5*S*)-3,5-dihydroxy-6-heptenoic acid and an appropriate water-soluble calcium salt, preferably from calcium chloride.

The starting sodium salt can be obtained according to the above-mentioned patent from the methyl ester of (*E*)-7-[4-(4-fluorophenyl)-6-isopropyl-2-[methyl(methylsulfonyl)amino]-pyrimidin-5-yl](3*R*,5*S*)-3,5-dihydroxy-6-heptenoic acid of formula II via hydrolysis with ethanolic sodium hydroxide or lately (according to international patent application WO 00/49014) from *tert*-butyl (*E*)-(6-[2-[4-(4-fluorophenyl)-6-isopropyl-2-[methyl-(methylsulfonyl)amino]pyrimidin-5-yl]vinyl](4*R*,6*S*')-2,2-dimethyl-[1,3]dioxan-4-yl)-acetate of formula III

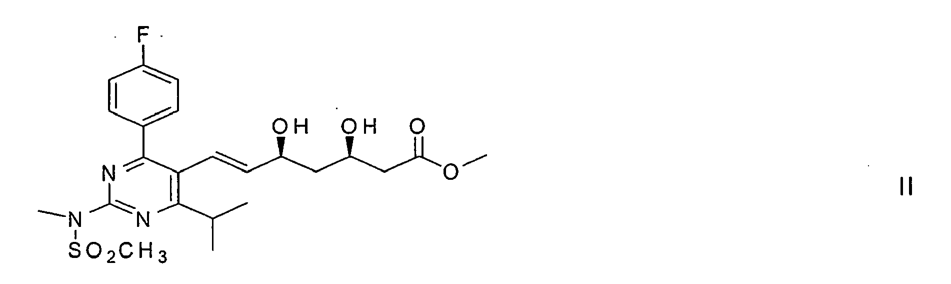

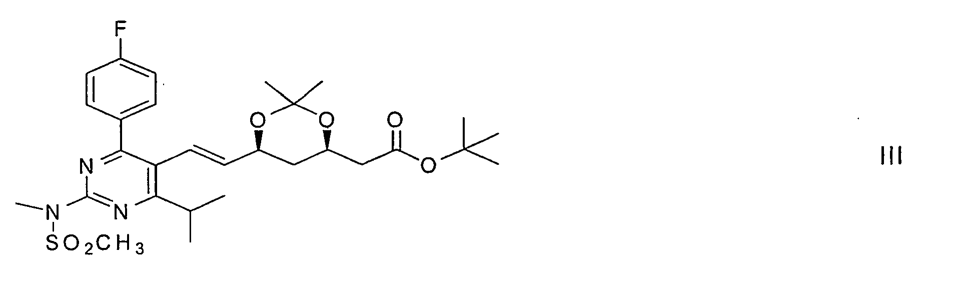

This intermediate product is first transferred to the corresponding sodium salt by consecutive stirring first with hydrochloric acid and then with sodium hydroxide. The calcium salt is subsequently obtained via addition of calcium chloride to the solution of the sodium salt in water. However, the salt prepared in this way is contaminated with inorganic substances. For example, residual sodium hydroxide reacts with calcium chloride to produce water-insoluble calcium hydroxide. Authors of the new patent application (WO 00/042024) assert that the substance prepared according to patent EP 521471 had an amorphous structure; nevertheless the process of its preparation was difficult to reproduce.

According to another patent application (WO 03/016317), the calcium salt can be obtained also via reaction of calcium hydroxide with lactone of formula IV 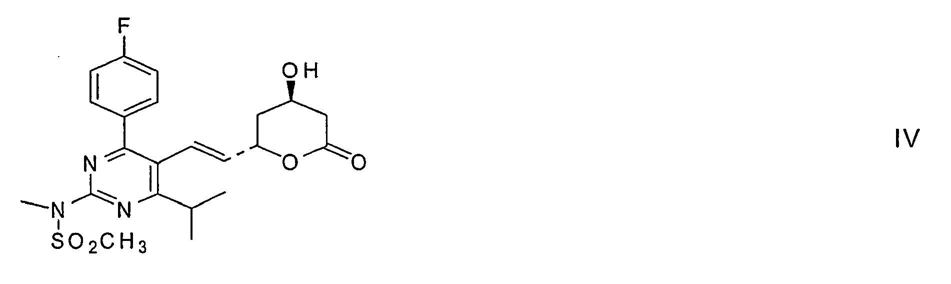 or other esters of rosuvastatin.

The objective of this invention is to describe a new, improved method of preparation of the hemi-calcium salt of (*E*)-7-[4-(4-fluorophenyl)-6-isopropyl-2-[methyl(methylsulfonyl)-amino]pyrimidin-5-yl](3*R*,5*S*)-3,5-dihydroxy-6-heptenoic acid (rosuvastatin), which would not have the mentioned disadvantages, and also an improved method of preparation of the amorphous form.

### Disclosure of Invention

The subject matter of the invention consists in an improved method of preparation of the hemi-calcium salt of (*E*)-7-[4-(4-fluorophenyl)-6-isopropyl-2-[methyl(methylsulfonyl)-amino]pyrimidin-5-yl](3*R*,5*S*)-3,5-dihydroxy-6-heptenoic acid of formula I, wherein an aqueous solution of the sodium or potassium salt of (*E*)-7-[4-(4-fluorophenyl)-6-isopropyl-2-[methyl(methylsulfonyl)amino]pyrimidin-5-yl](3*R*,5*S*)-3,5-dihydroxy-6-heptenoic acid, with optional admixture of sodium or potassium hydroxide or other sodium or potassium salts having inorganic anions, is extracted with an organic solvent, incompletely miscible with water, selected from the series of R¹COOR², R¹COR² and R¹OH, wherein R¹ and R² independently represent hydrogen or a residue of a C₁-C₁₀ aliphatic hydrocarbon, C₆ aromatic hydrocarbon, C₅ or C₆ cyclic hydrocarbon, or a combination of an aliphatic and aromatic or cyclic hydrocarbon, the extract being subsequently shaken with an aqueous solution of an inorganic or C₁-C₅ organic calcium salt, and the product of formula I is further isolated by cooling and/or adding an anti-solvent and filtration.

The aqueous solution of the sodium or potassium salt of (*E*)*-*7-[4-(4-fluorophenyl)-6-isopropyl-2-[methyl(methylsulfonyl)amino]pyrimidin-5-yl](3*R*,5*S*)-3,5-dihydroxy-6-heptenoic acid is preferably obtained stepwise by acidic hydrolysis and subsequent alkaline hydrolysis of the protected ester of formula III 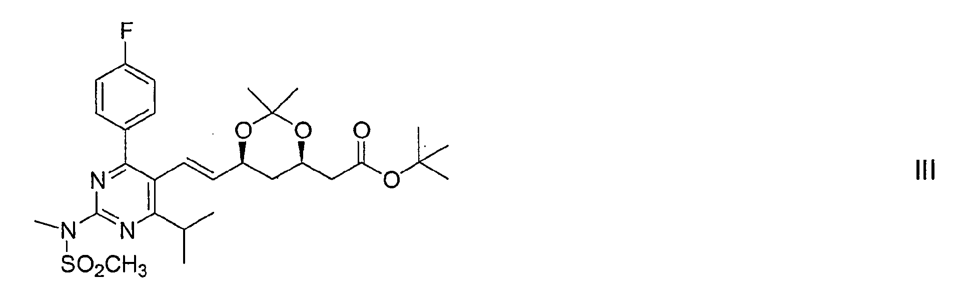 or by alkaline opening of the lactone of formula IV

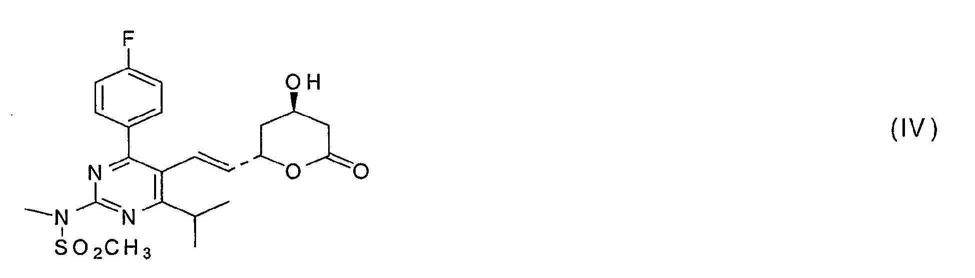

Extraction of the sodium or potassium salt from the aqueous solution is performed with an ester of formula R¹COOR², wherein R¹ and R² have the above mentioned meanings, or, even more preferably, extraction is made with ester R^{1'}COOR^{2'}, wherein R^{1'} and R^{2'} are independently hydrogen or a C₁-C₅ aliphatic residue, preferably with ethyl acetate.

This whole procedure is based on the surprising finding that the sodium or potassium salt of (*E*)-7-[4-(4-fluorophenyl)-6-isopropyl-2-[methyl(methylsulfonyl)amino]pyrimidin-5-yl](3*R*,5*S*)-3,5-dihydroxy-6-heptenoic acid can be quantitatively extracted from the aqueous phase into solvents of the type of esters, ketones or alcohols of formulae R¹COOR², R¹COR² or R¹OH, wherein R¹ and R² have the above-mentioned meaning. The sodium or potassium salt obtained in this way can be quantitatively transferred into the calcium salt by stirring with an aqueous solution of an inorganic or organic calcium salt. Rosuvastatin can be subsequently obtained by evaporation and crystallization.

Another aspect of the invention consists in a new method of preparation of the amorphous form, which is based on dissolving the calcium salt of rosuvastatin in a suitable solvent and adding the same to an anti-solvent, in which rosuvastatin is completely insoluble or little soluble. A solution of the hemi-calcium salt of rosuvastatin in an organic solvent selected from the series of R¹COOR², R¹COR² or R¹OH, wherein R¹ and R² have the above-mentioned meaning, is added dropwise to an anti-solvent in which rosuvastatin is insoluble, selected from the series including compounds of formulae R¹H and R¹OR², wherein R¹ and R² have the above-mentioned meaning, or water.

The compound of formula I is dissolved in a solvent preferably selected from the series of R^{1'}COOR^{2'}, R^{1'}COR^{2'} or R^{1'}OH, wherein R^{1'} and R^{2'} have the above-mentioned meanings, added dropwise to an anti-solvent in which rosuvastatin is insoluble, selected from the series including compounds of formulae R^{1'}H, R^{1'}OR^{2'}, wherein R^{1'} and R^{2'} have the above-mentioned meanings, or water.

The compound of formula I is preferably dissolved in a solution including ketones, particularly acetone, ethyl methyl ketone, isopropyl methyl ketone, alcohols, particularly methanol, ethanol, isopropanol, or butanols, and further esters, particularly of formic acid, acetic acid or propionic acid with methyl, ethyl or propyl alcohol, and the product is precipitated with solvents including heptane, pentane, cyclohexane, toluene, petroleum ether, diethyl ether or water.

### Brief Description of Drawings

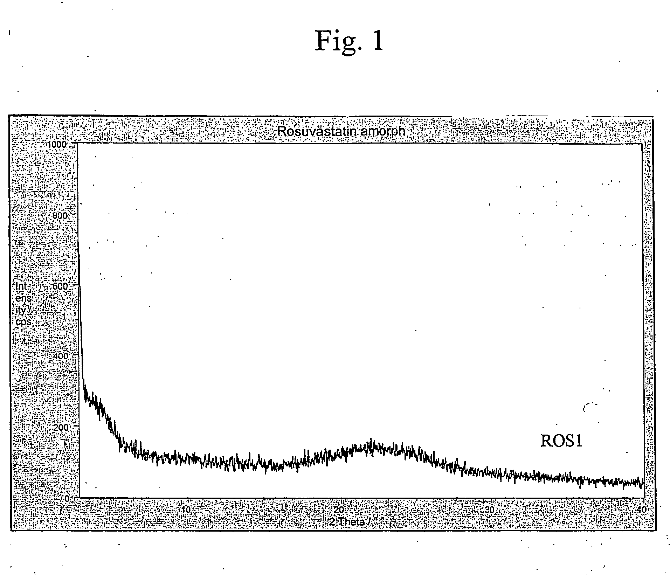

Figure 1 shows the diffraction pattern of an amorphous sample of the hemi-calcium salt of rosuvastatin.

### Detailed description of the invention

Esters of rosuvastatin or rosuvastatin lactone of formula IV can be hydrolyzed in aqueous tetrahydrofuran with sodium hydroxide and the resulting sodium salt of rosuvastatin can be quantitatively extracted into the organic phase, preferably with ethyl acetate. The sodium salt obtained in this way is converted into the calcium salt by shaking a solution of the sodium salt in ethyl acetate or another solvent of the above-mentioned type with a water soluble calcium salt, preferably calcium acetate. The residual inorganic contaminants are subsequently removed by washing with demineralized water. Evaporation and crystallization can produce rosuvastatin, which is not contaminated with inorganic substances.

According to the original patent EP 521471, the prepared rosuvastatin had an amorphous structure, but the process is not reproducible. The amorphous form has usually different dissolution characteristics and bio-availability than crystalline forms (Konno T.: *Chem. Pharm. Bull.* **1990,** *38,* 2003). In case of rosuvastatin, which is little soluble in water, it is important to have a reproducible process for obtaining the amorphous form.

In our method, it has turned out that perfectly amorphous rosuvastatin can be obtained by dissolving crystalline or semi-crystalline rosuvastatin in a solvent in which rosuvastatin is soluble under cold conditions or at increased temperatures, selected from the series of R¹COOR², R¹COR² or R¹OH, wherein R¹ and R² have the above-mentioned meaning, and by adding the resulting solution to an anti-solvent in which rosuvastatin is insoluble, selected from the series of R¹H, R¹OR², wherein R¹ and R² have the above-mentioned meaning, or water. The solvents in which rosuvastatin is soluble under cold conditions or at increased temperatures include those solvents in which solubility is higher than 1 g in 50 ml. Mixtures of suitable solvents can be also used. Examples of such preferable solvents include methanol, ethyl methyl ketone or ethyl acetate. The anti-solvents in which rosuvastatin is insoluble include those in which 1g of the substance does not dissolve in 1,000 ml of the solvent under cold conditions. Examples of such solvents include preferably hexane, pentane, diethyl ether or water. A more detailed list of these solvents has been presented above. The diffraction pattern of a perfectly amorphous sample (prepared according to Example 5) is shown in Fig. 1; the measurements were performed on diffractometer SEIFERT 3000 XRD with a graphite monochromator, radiation CoKα (λ = 1.790Å) within the range 2.5 - 40°2θ with a step 0.03.

The invention is elucidated in more detail in the following examples. The examples, which illustrate preferred alternatives of production of rosuvastatin according to the invention, have a purely illustrative character and do not limit the extent of the invention in any respect. Semi-crystalline rosuvastatin used in Example 5 was obtained according to the original patent EP 521471. Crystalline rosuvastatin used in Examples 6 and 7 was obtained according to WO 00/042024.

### Examples

### Example 1

Tetrahydrofuran (75 ml) is added to lactone IV (5 g, 10.8 mmol). A solution of 40% NaOH (10 ml) is added during 5 minutes to the solution obtained in this way and the formed heterogeneous mixture is vigorously stirred for 17 h and then poured into a separating funnel containing demineralized water (150 ml) and hexane (50 ml). After shaking, the organic layer is separated and the aqueous layer is extracted with a mixture of hexane (40 ml) and tetrahydrofuran (10 ml). After complete separation, the aqueous layer is extracted with ethyl acetate (1 x 40 ml, 3 x 20 ml). The ethyl acetate extract is then gradually shaken 3 times with demineralized water (5 ml), each containing 1 g of calcium acetate in 5 ml of water. The resulting ethyl acetate extract is washed with demineralized water (2 x 5 ml) and, after drying, is concentrated in a vacuum evaporator to a volume of 30 ml and added dropwise to hexane (150 ml) to give, after filtration, 4.5 g of amorphous rosuvastatin.
¹H NMR (DMSO) δ:
1.22 (d, J = 7, 6H); 1.41 (m, 1H); 1.61 (m, 1H); 2.18 (dd, J = 3, 2H); 3.43 (m, 1H); 3.45 (s, 3H); 3.57 (s, 3H); 3.83 (m,1H); 4.25(m, 1H); 5.56 (dd, J = 7.16, 1H); 6.58 (d, J =16, 1H); 7.33 (m, 2H); 7.76 (m, 2H)
MS for C₂₂H₂₈FN₃O₆SNa [M + Na]⁺: calculated 504.1; found 503.8.

### Example 2

Following the procedure described in Example 1 using potassium hydroxide instead of sodium hydroxide for the hydrolysis of the ester, the corresponding potassium salt of rosuvastatin is obtained. The solution is further treated according to the procedure described in Example 1, to provide 4.2 g of amorphous rosuvastatin.

### Example 3

Tetrahydrofuran (15 ml) is added to ester III (1 g, 1.7 mmol) and after a clear solution is formed, 10% HCl (4 ml) is added. The mixture is stirred for additional 24 hours at ambient temperature. Then, a solution of 40 % NaOH (2 ml) is added to the solution during 5 min and the formed heterogeneous mixture is vigorously stirred for 17 h and then poured into a separating funnel containing demineralized water (30 ml) and hexane (10 ml). After shaking, the organic layer is separated and the aqueous layer is extracted with a mixture of hexane (8 ml) and tetrahydrofuran (2 ml). After complete separation, the aqueous layer is extracted with ethyl acetate (1 x 20 ml, 3 x 10 ml). Combined ethyl acetate extracts are gradually shaken 3 times with demineralized water (1 ml), each containing 0.2 g of calcium acetate in 1 ml of water. The resulting ethyl acetate solution is washed with demineralized water (2 x 3 ml) and after drying with calcium sulfate, it is evaporated in a vacuum evaporator. After crystallization from acetonitrile and water, 0.7 g of rosuvastatin is obtained.

### Example 4

Tetrahydrofuran (15 ml) is added to ester II (1 g, 2 mmol) and after complete dissolution, a solution of 40 % NaOH (2 ml) is added to the solution over 5 min and the formed heterogeneous mixture is vigorously stirred for 17 h and then poured in a separating funnel containing demineralized water (30 ml) and hexane (10 ml). After shaking, the organic layer is separated and the aqueous layer is extracted with a mixture of hexane (8 ml) and tetrahydrofuran (2 ml). After complete separation, the aqueous layer is extracted with ethyl acetate (1 x 20 ml, 3 x 10 ml). The ethyl acetate solution is subsequently shaken 3 times with demineralized water (1 ml), each containing 0.2 g of calcium acetate in 1 ml of water. The resulting ethyl acetate solution is washed with demineralized water (2 x 3 ml) and evaporated in a vacuum evaporator. After crystallization from acetonitrile and water, 0.7 g of rosuvastatin is obtained.

### Example 5

Semi-crystalline rosuvastatin (1 g) is dissolved in ethyl methyl ketone (10 ml) at 40 °C. After being filtered, the resulting solution is added dropwise to pentane (70 ml), while the mixture is vigorously stirred. After 30 min of stirring, the solution is sucked off and dried in vacuo to give 0.95 g of amorphous rosuvastatin.

### Example 6

Crystalline rosuvastatin (1.5 g) is dissolved in methanol (10 ml) at 25 °C. After being filtered, the resulting solution is added dropwise to water (150 ml), while the mixture is vigorously stirred at 5 °C. After 30 min of stirring, the solution is sucked off and dried in vacuo to give 1.3 g of amorphous rosuvastatin.

### Example 7

Crystalline rosuvastatin (1 g) is dissolved in methanol (10 ml) at 25 °C. After being filtered, the resulting solution is added dropwise to diethyl ether (150 ml) at 25 °C. After 30 min of stirring, the solution is sucked off and dried in vacuo to give 0.7 g of amorphous rosuvastatin.

## Claims

1. A method of preparation of the hemi-calcium salt of (*E*)-7-[4-(4-fluorophenyl)-6-isopropyl-2-[methyl(methylsulfonyl)amino]pyrimidin-5-yl](3*R*,5*S*)-3,5-dihydroxy-6-heptenoic acid of formula I, i.e. rosuvastatin 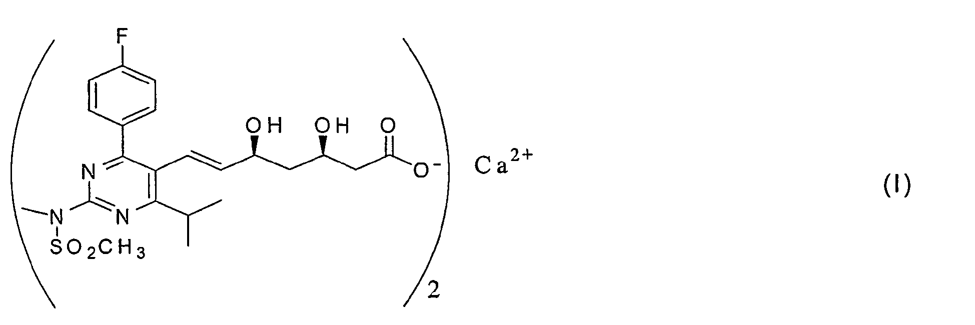 **characterized in that** an aqueous solution of the sodium or potassium salt of (*E*)-7-[4-(4-fluorophenyl)-6-isopropyl-2-[methyl(methylsulfonyl)amino]pyrimidin-5-yl](3*R*,5*S*)-3,5-dihydroxy-6-heptenoic acid, with optional admixture of sodium or potassium hydroxide or other sodium or potassium salts having inorganic anions, is extracted with an organic solvent, incompletely miscible with water, selected from the series of R¹COOR², R¹COR² and R¹OH, wherein R¹ and R² independently represent hydrogen or a residue of a C₁-C₁₀ aliphatic hydrocarbon, C₆ aromatic hydrocarbon, C₅ or C₆ cyclic hydrocarbon, or a combination of an aliphatic and aromatic or cyclic hydrocarbon, the extract being subsequently shaken with an aqueous solution of an inorganic or C₁-C₅ organic calcium salt, and the product of formula I is further isolated by cooling and/or adding an anti-solvent and filtration, and, optionally, it is converted into its amorphous form.

2. The method according to claim 1 **characterized in that** the aqueous solution of the sodium or potassium salt of (*E*)-7-[4-(4-fluorophenyl)-6-isopropyl-2-[methyl-(methylsulfonyl)amino]pyrimidin-5-yl](3*R*,5*S*)-3,5-dihydroxy-6-heptenoic acid is obtained stepwise by acidic hydrolysis and subsequent alkaline hydrolysis of the protected ester of formula III 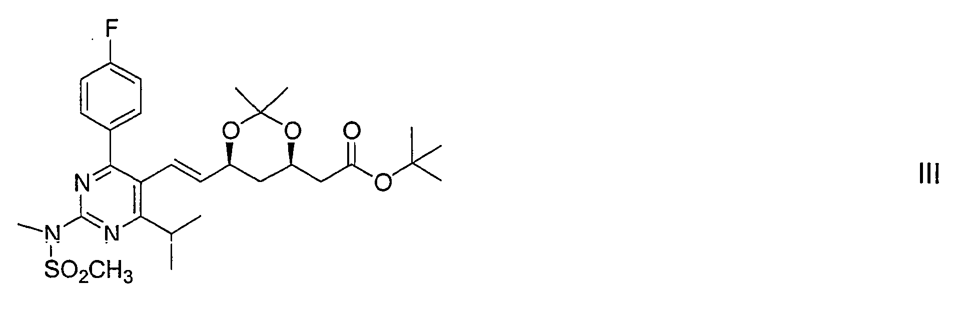 or by alkaline opening of the lactone of formula IV

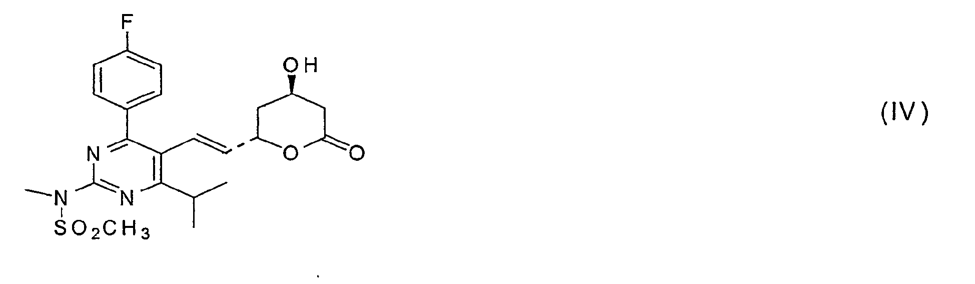

3. The method according to claim 1 **characterized in that** the extraction of the sodium or potassium salt from the aqueous solution is performed with an ester of formula R¹COOR², wherein R¹ and R² are as defined in claim 1.

4. The method according to claim -1 **characterized in that** the extraction is performed with ester R^{1'}COOR^{2'}, wherein R^{1'} and R^{2'} are independently hydrogen or a C₁-C₅ aliphatic residue, preferably with ethyl acetate.

5. A method of the preparation of the amorphous form of the hemi-calcium salt of (*E*)-7-[4-(4-fluorophenyl)-6-isopropyl-2-[methyl(methylsulfonyl)amino]pyrimidin-5-yl](3*R*,5*S*)-3,5-dihydroxy-6-heptenoic acid of formula I, i.e. rosuvastatin, according to claim 1, **characterized in that** a solution of the hemi-calcium salt of (*E*)-7-[4-(4-fluorophenyl)-6-isopropyl-2-[methyl(methylsulfonyl)amino]pyrimidin-5-yl](3*R*,5*S*)-3,5-dihydroxy-6-heptenoic acid in an organic solvent selected from the series of R¹COOR², R¹COR² and R¹OH, wherein R¹ and R² are as defined in claim 1, is added dropwise to a solvent in which rosuvastatin is insoluble, selected from the series including compounds of formulae R¹H and R¹OR², wherein R¹ and R² are as defined in claim 1, and water.

6. The method according to claim 5 **characterized in that** the compound of formula I is dissolved in a solvent selected from the series of R^{1'}COOR^{2'}, R^{1'}COR^{2'} and R^{1'}OH, wherein R^{1'} and R^{2'} are as defined in claim 4, is added dropwise to a solvent in which rosuvastatin is insoluble, selected from the series including compounds of formulae R^{1'}H or R^{1'}OR^{2'}, wherein R^{1'} and R²' are as defined in claim 4, and water.

7. The method according to claim 5 **characterized in that** the compound of formula I is dissolved in a solvent including ketones, particularly acetone, ethyl methyl ketone, isopropyl methyl ketone, alcohols, particularly methanol, ethanol, isopropanol, or butanols, further esters, particularly of formic acid, acetic acid or propionic acid with methyl, ethyl or propyl alcohol, and the product is precipitated with solvents including heptane, pentane, cyclohexane, toluene, petroleum ether, diethyl ether or water.

## Patentansprüche

1. Verfahren zur Herstellung des Hemicalciumsalzes von (*E*)-7-[4-(4-Fluorphenyl)-6-isopropyl-2-[methyl(methylsulfonyl)amino]pyrimidin-5-yl](3*R*,5*S*)-3,5-dihydroxy-6-heptensäure der Formel I, d.h. Rosuvastatin 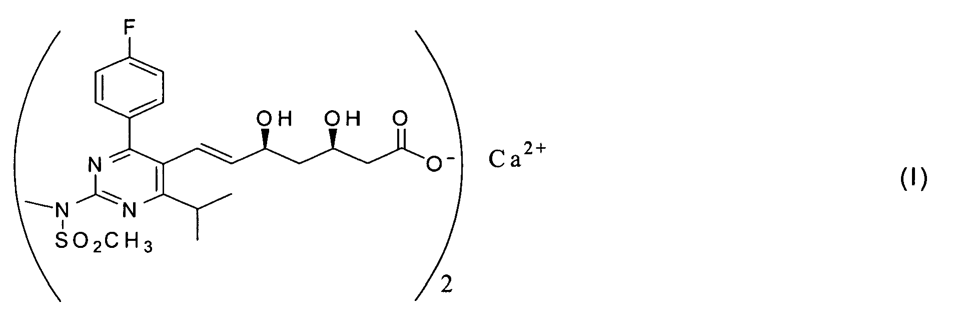 **dadurch gekennzeichnet, dass** die wässrige Lösung des Natrium- oder Kaliumsalzes von (E)-7-[4-(4-Fluorphenyl)-6-isopropyl-2-[methyl(methylsulfonyl)amino]pyrimidin-5-yl](3R,5S)-3,5-dihydroxy-6-heptensäure mit eventueller Beimischung vom Natrium- oder Kaliumhydroxid oder weiteren Natrium- oder Kaliumsalzen mit anorganischem Anion mit einem mit Wasser beschränkt mischbaren organischen Lösungsmittel extrahiert wird, welches ausgewählt ist aus der Reihe von R¹COOR², R¹COR² und R¹OH, wobei R¹ a R² unabhängig Wasserstoff oder einen aliphatischen C₁ bis C₁₀ Kohlenwasserstoffrest, aromatischen C₆ Kohlenwasserstoffrest, zyklischen C₅ oder C₆ Kohlenwasserstoffrest, oder eine Kombination vom aliphatischen und aromatischen oder zyklischen Kohlenwasserstoffresten darstellen, der Extrakt ist weiterhin mit einer wässrigen Lösung eines anorganisches oder C₁ bis C₅ organisches Kalziumsalzes umgeschüttelt und das Produkt der Formel I ferner mit Abkühlung und/oder Zugabe eines Anti-Lösungsmittels und Filtration isoliert und eventuell in seine amorphe Form transformiert wird.

2. Verfahren nach Anspruch 1, **dadurch gekennzeichnet, dass** die wässrige Lösung des Natrium- oder Kaliumsalzes von (E)-7-[4-(4-Fluorphenyl)-6-isopropyl-2-[methyl(methylsulfonyl)amino]pyrimidin-5-yl](3R,5S)-3,5-dihydroxy-6-heptensäure stufenweise durch Säurehydrolyse und nachfolgend durch alkalische Hydrolyse des geschützten Ester der Formel III 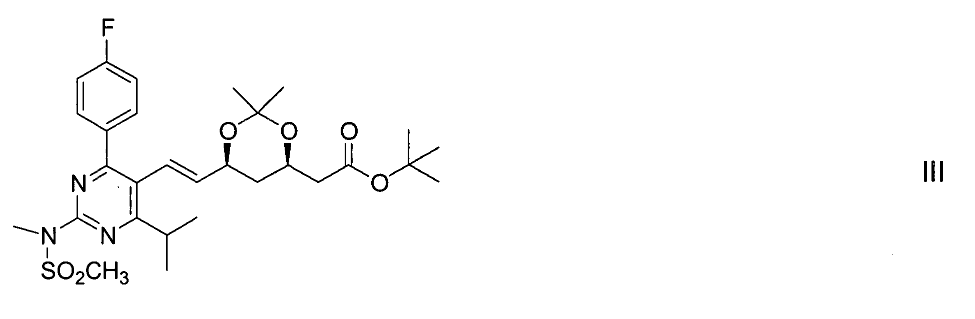 oder durch alkalische Öffnung vom Lakton der Formel IV 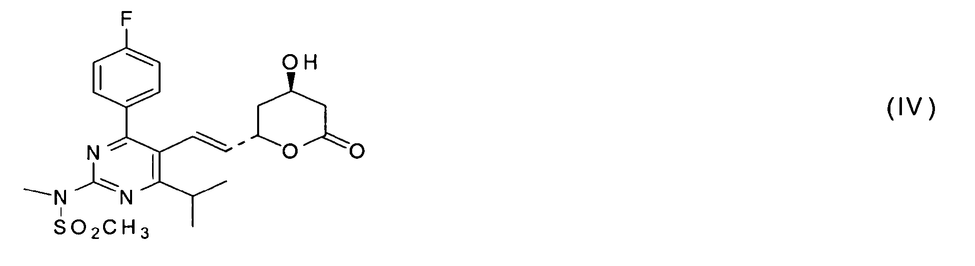 gewonnen wird.

3. Verfahren nach Anspruch 1, **dadurch gekennzeichnet, dass** die Extraktion des Natrium- oder Kaliumsalzes aus der wässrigen Lösung mit einem Ester der Formel R¹COOR², wobei R¹ und R² von der gleichen Bedeutung wie im Anspruch 1 sind, durchgeführt wird.

4. Verfahren nach Anspruch 1, **dadurch gekennzeichnet, dass** die Extraktion mit dem Ester R^{1'}COOR^{2'}, wobei R^{1'} und R^{2'} unabhängig Wasserstoff oder ein C₁ bis C₅ aliphatischer Rest sind, vorzugsweise mit Äthylacetat, durchgeführt wird.

5. Verfahren zur Herstellung der amorphen Form des Hemicalciumsalzes von (*E*)-7-[4-(4-Fluorphenyl)-6-isopropyl-2-[methyl(methylsulfonyl)amino]pyrimidin-5-yl](3*R*,5*S*)-3,5-dihydroxy-6-heptensäure der Formel I, d.h. Rosuvastatin, nach Anspruch 1, **dadurch gekennzeichnet, dass** die Lösung des Hemicalciumsalzes von (E)-7-[4-(4-Fluorphenyl)-6-isopropyl-2-[methyl(methylsulfonyl)amino]pyrimidin-5-yl](3*R*,5*S*)-3,5-dihydroxy-6-heptensäure in einem organischen Lösungsmittel, welches ausgewählt ist aus der Reihe von R¹COOR², R¹COR² und R¹OH, wobei R¹ und R² von der gleichen Bedeutung wie im Anspruch 1 sind, in ein Lösungsmittel, worin Rosuvastatin unlöslich ist, welches ausgewählt ist aus der Reihe umfassend die Verbindungen der Formeln R¹H und R¹OR², wobei R¹ und R² von der gleichen Bedeutung wie im Anspruch 1 sind, und Wasser, tropfenweise zugegeben wird.

6. Verfahren nach Anspruch 5, **dadurch gekennzeichnet, dass** die Verbindung der Formel I in einem Lösungsmittel, welches ausgewählt ist aus der Reihe von R^{1'}COOR^{2'}, R^{1'}COR^{2'} und R^{1'}OH, wobei R^{1'} und R^{2'} von den im Anspruch 4 angeführten Bedeutungen sind, gelöst wird und in ein Lösungsmittel, worin Rosuvastatin unlösbar ist, welches ausgewählt ist aus der Reihe umfassend die Verbindungen der Formel R^{1'}H und R^{1'}OR^{2'}, wobei R^{1'} und R^{2'} von der Bedeutung angeführt im Anspruch 4 sind, und das Wasser, tropfenweise zugegeben wird.

7. Verfahren nach Anspruch 5, **dadurch gekennzeichnet, dass** die Verbindung der Formel I in einem Lösungsmittel umfassend Ketone, insbesondere Aceton, Äthylmethylketon, Isopropylmethylketon, Alkohole, insbesondere Methanol, Äthanol, Isopropanol, oder Butanols, ferner Ester, insbesondere von Ameisensäure, Essigsäure oder Propionsäure mit Methyl-, Äthyl- oder Propylalkohol, gelöst wird und das Produkt mit Lösungsmitteln umfassend Heptan, Pentan, Cyclohexan, Toluol, Petroläther, Diäthyläther oder Wasser ausgefallen wird.

## Revendications

1. Procédé de préparation du sel hémicalcique de l'acide (*E*)-7-[4-(4-fluorophényl)-6-isopropyl-2-[méthyl(methylsulfonyl)amino]pyrimidin-5-yl](3*R*,5*S*)- 3,5-dihydroxy-6-heptenoïque de formule I, c'est-à-dire de la rosuvastatine 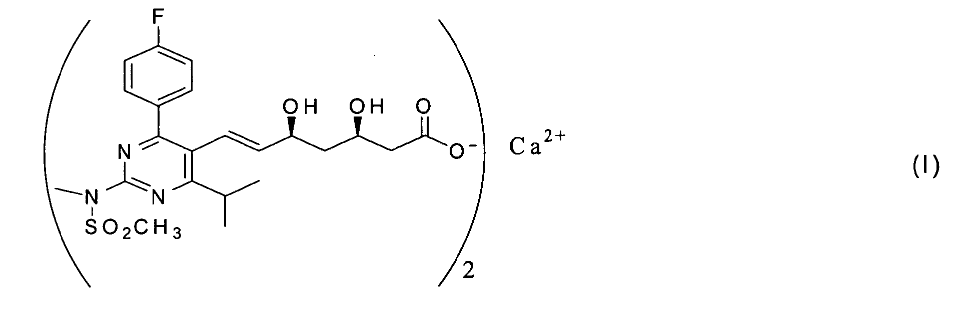 **caractérisé en ce qu'**une solution aqueuse du sel de sodium ou de potassium de l'acide (E)-7-[4-(4-fluorophényl)-6-isopropyl-2-[méthyl(methylsulfonyl)amino]pyrimidin-5-yl](3R,5S)-3,5-dihydroxy-6-heptenoïque, avec une addition éventuelle d'hydroxyde de sodium ou de potassium ou d'autres sels de sodium ou de potassium avec l'anion inorganique, est extrait par un solvant organique, miscible avec de l'eau de manière limitée, choisi parmi R¹COOR², R¹COR² et R¹OH, où R¹ et R² signifient de manière indépendante l'hydrogène ou un résidu d'une hydrocarbure aliphatique C₁ à C₁₀, d'une hydrocarbure aromatique C₆, d'une hydrocarbure cyclique C₅ ou C₆, ou une combinaison d'une hydrocarbure aliphatique et aromatique ou cyclique, l'extrait étant puis agité avec une solution aqueuse d'un sel de calcium inorganique ou organique C₁ à C₅, et le produit de formule I est ensuite isolé par refroidissement et/ou adjonction d'un anti-solvant et par filtration et, le cas échéant, est transformé en sa forme amorphe.

2. Procédé selon la revendication 1, **caractérisé en ce que** la solution aqueuse du sel de sodium ou de potassium de l'acide (E)-7-[4-(4-fluorophényl)-6-isopropyl-2-[méthyl(methylsulfonyl)amino]pyrimidin-5-yl](3R,5*S*)-3,5-dihydroxy-6-heptenoïque est obtenue graduellement par hydrolyse acide et puis par hydrolyse alcaline de l'ester protégé de formule III 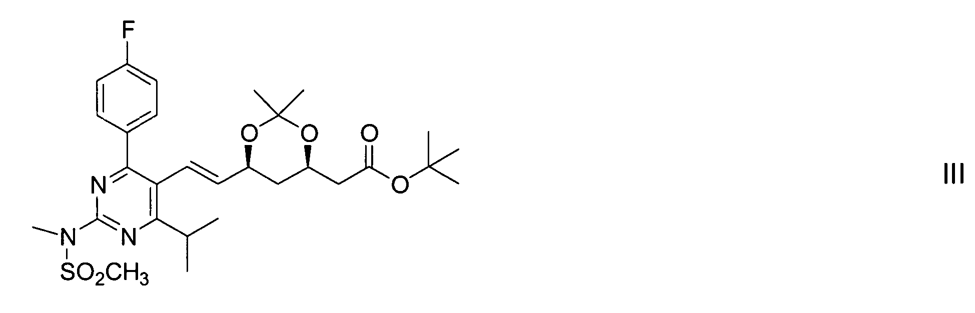 ou par ouverture alcaline de la lactone de formule IV

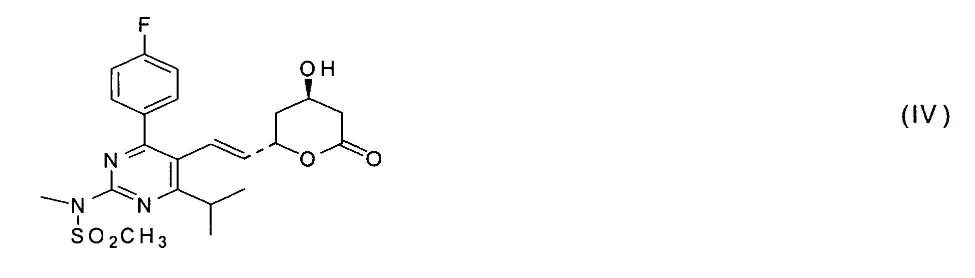

3. Procédé de préparation selon la revendication 1, **caractérisé en ce que** l'extraction du sel de sodium ou de potassium de la solution aqueuse est effectuée par un ester de formule R¹COOR², dans laquelle R¹ et R² ont la même signification que dans la revendication 1.

4. Procédé de préparation selon la revendication 1, **caractérisé en ce que** l'extraction est effectuée par l'ester R^{1'}COOR^{2'}, où R^{1'} et R²' sont de manière indépendante l'hydrogène ou un résidu aliphatique C₁ à C₅, préférentiellement par l'acétate d'éthyle.

5. Procédé de préparation de la forme amorphe du sel hémicalcique de l'acide (*E*)-7-[4-(4-fluorophényl)-6-isopropyl-2-[méthyl(methylsulfonyl)amino]pyrimidin-5-yl](3*R*,5*S*)-3,5-dihydroxy-6-heptenoïque de formule I, c'est-à-dire de la rosuvastatine, selon la revendication 1, **caractérisé en ce que** une solution du sel hémicalcique de l'acide (*E*)-7-[4-(4-fluorophényl)-6-isopropyl-2-[méthyl(methylsulfonyl)amino]-pyrimidin-5-yl](3*R*,5*S*)-3,5-dihydroxy-6-heptenoïque dans un solvant organique choisi parmi R¹COOR², R¹COR² et R¹OH, où R¹ et R² ont la même signification que dans la revendication 1, est ajoutée goutte à goutte à un solvant dans lequel la rosuvastatine est insoluble, choisi parmi les composés des formules R¹H et R¹OR², dans lesquelles R¹ et R² ont la signification mentionnée à la revendication 1, et l'eau.

6. Procédé de préparation selon la revendication 5, **caractérisé en ce que** le composé de formule I est dissout dans un solvant choisi parmi R^{1'}COOR^{2'}, R^{1'}COR^{2'} et R^{1'}OH, où R^{1'} et R^{2'} ont les significations mentionnées dans la revendication 4, et ajouté goutte à goutte à un solvant dans lequel la rosuvastatine est insoluble, choisi parmi les composés des formules R^{1'}H et R^{1'}OR^{2'}, dans lequelles R^{1'} et R^{2'} ont la signification mentionnée à la revendication 4, et l'eau.

7. Procédé de préparation selon la revendication 5, **caractérisé en ce que** le composé de formule I est dissout dans un solvant incluant des cétones, notamment l'acétone, l'éthylméthylcétone, l'isopropylméthylcétone, des alcools, notamment le méthanol, l'éthanol, l'isopropanol, ou des butanols, ou encore des esters, notamment ceux des acides formique, acétique ou propionique avec l'alcool méthylique, éthylique ou propylique, et le produit est précipité avec des solvants incluant l'heptane, le pentane, le cyclohexane, le toluène, l'éther de pétrole, l'éther éthylique ou l'eau.
